(19) Europäisches Patentamt / European Patent Office / Office européen des brevets

(11) EP 2 121 716 B1

(12) **EUROPEAN PATENT SPECIFICATION**

(45) Date of publication and mention of the grant of the patent:
**09.12.2015 Bulletin 2015/50**

(21) Application number: **08724557.7**

(22) Date of filing: **17.01.2008**

(51) Int Cl.:
***C07H 17/08*** (2006.01) ***A61K 47/10*** (2006.01)
***A61K 9/20*** (2006.01) ***A61K 31/335*** (2006.01)
***A61P 31/04*** (2006.01) ***A61P 1/12*** (2006.01)

(86) International application number:
**PCT/US2008/000591**

(87) International publication number:
**WO 2008/091518 (31.07.2008 Gazette 2008/31)**

(54) **COMPOSITIONS OF STABLE TIACUMICINS**

ZUSAMMENSETZUNGEN AUS STABILEN TIACUMICINEN

COMPOSITIONS DE TIACUMICINES STABLES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **19.01.2007 US 881137 P**

(43) Date of publication of application:
**25.11.2009 Bulletin 2009/48**

(73) Proprietor: **Merck Sharp & Dohme Corp.**
**Rahway, NJ 07065 (US)**

(72) Inventors:
- **SANGHVI, Suketu**
  **Kendall Park, NJ 08824 (US)**
- **ROACH, Mark**
  **Scarsdale, NY 10583 (US)**
- **ZHOU, Joseph, F.**
  **Denville, NJ 07834 (US)**
- **HE, Ping**
  **Suffern, NJ 10901 (US)**
- **MITTLEBERG, Michael**
  **Spring Valley, NJ 10977 (US)**

(74) Representative: **Jaap, David Robert**
**MSD**
**Hertford Road**
**Hoddesdon, Herts EN11 9BU (GB)**

(56) References cited:
**WO-A1-2006/085838** **US-A1- 2004 224 020**
**US-A1- 2006 257 981** **US-A1- 2006 257 981**
**US-A1- 2006 269 485** **US-A1- 2006 269 485**

- **Revill P.,Serradell N., Bolos J.: "Tiacumicin B", Drugs of the future Internet Article, vol. 31, no. 6 31 December 2006 (2006-12-31), pages 494-497, XP002696172, Retrieved from the Internet: URL: http://journals.prous.com/journals/ser vlet/ xmlxsl/dof/20063106/pdf/df310494.pdf? p_ JournalId=2&p_refId=1000709&p_IsPs=N [retrieved on 2013-04-25]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 121 716 B1

## Description

### FIELD OF THE INVENTION

The invention relates generally to the field of medicinal formulations, and more particularly to methods of preparing pharmaceutical compositions of one or more tiacumicins as defined by the claims, such as difimicin, that are substantially stable to allow for increased shelf life and improved methods of treatment.

### BACKGROUND OF THE INVENTION

Tiacumicins are a family of structurally related compounds that contain an 18-membered macrolide ring. Members of the tiacumicin family (e.g., tiacumicin A-F) have been disclosed, for example, by U.S. Patent No. 4,918,174 and by J. Antibiotics, 1987, 575-888. Tiacumicins have been disclosed as having activity against a variety of bacterial pathogens. As such, tiacumicins are generally expected to be useful in the treatment of bacterial infections in mammals, and especially those of the gastrointestinal tract. Examples of such treatments include, but are not limited to, treatment of *Clostridium difficile-associated* diarrhea (CDAD), and other diseases, infections, and/or conditions, such as colitis, pseudomembranous colitis, antibiotic associated diarrhea, and infections due to *C. difficile, C. perfringens, Staphylococcus* species, such as methicillin-resistant *Staphylococcus aureus* (MRSA), *Enterococcus,* such as vancomycin-resistant enterococci (VRE), and similar diseases, including but not limited to clostridial enterocolitis, neonatal diarrhea, antibiotic-associated enterocolitis, sporadic enterocolitis, nosocomial enterocolitis, colitis membranous, infectious diarrhea, and irritable bowel syndrome. See, for example, WO2006/085838, WO 2005/112990, US2006/0100164, and Swanson et al., "In vitro and in vivo evaluation of tiacumicins B and C against Clostridium difficile", Antimicrobial Agents and Chemotherapy (June 1991) pp. 1108-1111.

Difimicin, also described as 3-[[[6-Deoxy-4-*O*-(3,5-dichloro-2-ethyl-4,6-dihydroxybenzoyl)-2-*O*-methyl-β-D-mannopyranosyl]oxy]-methyl]-12(*R*)-[[6-deoxy-5-*C*-methyl-4-*O*-(2-methyl-1-oxopropyl)-β-D-lyxohexopyranosyl]oxy]-11(*S*)-ethyl-8(*S*)-hydroxy-18(*S*)-(1(*R*)-hydroxyethyl)-9,13,15-trimethyloxacyclooctadeca-3,5,9,13,15-pentaene-2-one, is a narrow-spectrum antibiotic with the following general structure.

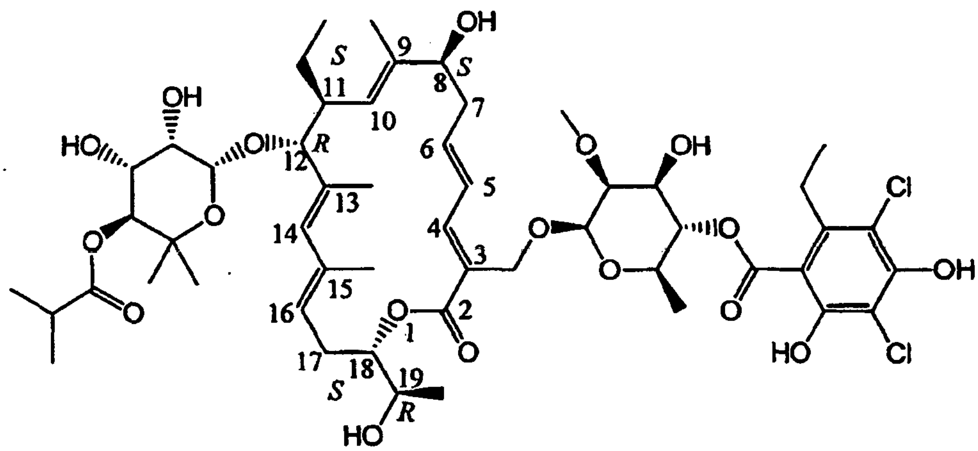

Processes for obtaining difimicin and derivatives thereof are disclosed, for example, in U.S. Patent Application Publication No. 2006/0257981, and in U.S. Patent Nos. 5,583,115 and 5,767,096.

As tiacumicins have been found to have poor flow properties and stability issues in the presence of humidity, compositions of these drugs that would be stable in the presence of humidity are highly desirable. The present invention satisfies this need for new formulations of tiacumicins, such as difimicin, with increased stability and shelf life.

US 2006/0269485 A1 discloses a therapeutic kit to provide a safe and effective dosage of an antibiotic agent, including an aerosol packaging assembly with the defined features. Revill et al, Drugs of the Future 2006, 31(6): 494-497 describes Tiacumicin B and the macrolide antibiotic treatment of *C.difficile* - Associated Diarrhoea.

US 2006/0257981 A1 discloses methods, processes and materials for the production and recovery of Tiacumicins produced by culturing a defined microorganism.

WO2006085838 discloses compositions comprising the C19R-enantiomer of difimicin in combination with pharmaceutical carriers like microcrystalline cellulose and the like.

## SUMMARY OF THE INVENTION

The present invention as defined by the claims relates to compositions that substantially increase the stability of difimicin and other tiacumicins. As such, embodiments of the present invention prevent decreases in the effective dosages of compositions of difimicin, and substantially increase shelf life of such compositions.

Embodiments of the present invention provide a pharmaceutical composition that is substantially stable, comprising a therapeutically effective amount of difimicin, a stabilizing amount of one or more antioxidants, preferably butylated hydroxytoluene, and optionally one or more pharmaceutically acceptable excipients. In some embodiments, the stabilizing amount of one or more antioxidants is from about 0.001% to about 50% of the total weight of said composition.

Embodiment of the present invention also provide a composition for use in the treatment or prevention of a disease, infection, and/or other condition associated with the use of antibiotics, cancer chemotherapies, or antiviral therapies, comprising administering a pharmaceutical composition that is substantially stable, preferably in the presence of heat and/or humidity, to a subject, comprising a therapeutically effective amount of difimicin, a stabilizing amount of one or more defined antioxidants, preferably butylated hydroxytoluene, and optionally one or more pharmaceutically acceptable excipients. Exemplary diseases, infections, and/or conditions include, but are not limited to the following: *C. difficile*-associated diarrhea (CDAD), colitis, pseudomembranous colitis, antibiotic associated diarrhea, infections due to *C. difficile, C. perfringens, Staphylococcus* species, or *Enterococcus,* clostridial enterocolitis, neonatal diarrhea, antibiotic-associated enterocolitis, sporadic enterocolitis, nosocomial enterocolitis, and irritable bowel syndrome. In a preferred embodiment, the disease, infection, and/or other condition is *C. difficile*-associated diarrhea (CDAD).

Some embodiments provide a pharmaceutical composition comprising a therapeutically effective amount of difimicin, butylated hydroxytoluene in an amount of about 0.001% to about 5% of the total weight of said composition, and optionally one or more of microcrystalline cellulose, starch, hydroxypropylcellulose, sodium starch glycolate, and magnesium stearate.

In some embodiments, difimicin is administered with related compound A, related compound B, related compound C, related compound D, related compound E, related compound F, related compound G, related compound H, related compound I, related compound J, related compound K, related compound L, related compound M, related compound N, related compound O, lipiarmycin A4, tiacumicin A, tiacumicin F, or tiacumicin C, combinations thereof, or all of these compounds.

Embodiments of the present invention also provide a pharmaceutical composition comprising a therapeutically effective amount of difimicin, butylated hydroxytoluene in an amount of about 0.001% to about 5% of the total weight of said composition, and optionally one or more of microcrystalline cellulose, starch, hydroxypropylcellulose, sodium starch glycolate, and magnesium stearate.

Other aspects, features, and advantages of the invention will become apparent from the following detailed description and figures.

## BRIEF DESCRIPTION OF THE FIGURES

Figure 1 shows possible structures of compounds related to difimicin.

Figure 2 shows a high performance liquid chromatography (HPLC) chromatogram at time zero of a formulation including difimicin but no antioxidant.

Figure 3 shows an HPLC chromatogram of a stressed tablet after two months at 40°C/75%RH having a formulation including difimicin but no antioxidant.

Figure 4 shows an HPLC chromatogram at time zero of a formulation including difimicin and BHT.

Figure 5 shows an HPLC chromatogram of a stressed tablet after two months at 40°C/75%RH having a formulation including difimicin and BHT.

## DETAILED DESCRIPTION OF THE INVENTION

Embodiments of the present invention include a pharmaceutical composition that is substantially stable comprising a therapeutically effective amount of difimicin, a stabilizing amount of one or more defined antioxidants, and optionally one or more pharmaceutically acceptable excipients.

As used herein, "substantially stable" means that the active ingredient has greater than or equal to about 90% of the assay of active ingredient initially present in the composition at time 0 at the stated conditions for at least about 6 months, preferably at least about 1 year, more preferably at least about 18 months, and most preferably at least about 2 years. Alternatively, a composition is "substantially stable" where the composition has an increase of not more than about 1.5% of related impurities to difimicin than initially present at time 0, preferably less than about 1.0%, more

preferably less than about 0.75%, and most preferably less than about 0.50%, after storage at the stated conditions for at least about 6 months, preferably at least about 1 year, more preferably at least about 18 months, and most preferably at least about 2 years. In preferred embodiments of the present invention, the pharmaceutical compositions are substantially stable in the presence of humidity and/or temperature changes ordinarily present for products in the pharmaceutical industry (e.g., during, but not limited to, manufacture, packaging, distribution, and/or storage by the manufacturers, distributors, and/or consumers) for about 1, 2, 3, or 6 months, preferably at least about 1 year, more preferably at least about 18 months, and most preferably at least about 2 years.

The term "related impurity" refers to an unwanted degradation product of the one or more tiacumicins, such as related compound L, a degradation product of difimicin.

Embodiments of the present invention are considered stable when stored at ambient storage conditions of about 18° C to about 30° C, preferably about 25° C. and up to about 60% relative humidity (RH) (e.g., at least about 20% RH, preferably at least about 30% RH, more preferably at least about 50% RH) for a period of at least about 1, 2, or 3 months, preferably at least about 6 months, more preferably at least about 1 year, even more preferably at least about 18 months, and most preferably at least about 2 years. Embodiments are also considered stable when stored at about 40 °C., most preferably at accelerated storage conditions of about 40° C. and up to about 75% RH (e.g., at least about 40% RH, preferably at least about 50% RH, more preferably at least about 60% RH, and most preferably about 75% RH) for a period of at least 3 months, preferably at least about 6 months, more preferably at least 1 year, even more preferably at least about 18 months, and most preferably at least about 2 years. Generally, a formulation tested as stable under accelerated storage conditions for three months will be stable under ambient storage conditions for at least about two years.

Stability of embodiments of the present invention may evaluated by any methods known to those of skill in the art. For example, stability may be evaluated through an HPLC assay and determination of chromatographic purity. The pharmaceutical compositions of FIG. 2-5 were evaluated using the following parameters, procedures, and calculations:

Mobile Phase A: Add 2.0 mL of trifluoroacetic acid to 2 L of HPLC water, filter and degas.

Mobile Phase B: Add 1.0 mL of trifluoroacetic acid to 2 L of acetonitrile, filter and degas.

Column: 4.6 x 150 mm column that contains octylsilane chemically bonded to porous silica or ceramic microparticles 3 to 10 μm in diameter (e.g. Agilent Zorbax Eclipse XDB-C8 3.5 μm, or equivalent).

Detector: 230 nm.

Flow Rate About 1.0 mL/min.

Injection Volume: About 10 μL.

Run Time: About 20 min.

pH 4 Citrate Buffer: Dissolve about 1.9 g of anhydrous citric acid in about 1000 mL of HPLC grade water, adjust pH to 4.0 ± 0.1 with 10 N NaOH.

Diluent: Mix 200 mL of pH 4 citrate buffer and 300 mL acetonitrile.

Gradient Program:

| Time (min) | % Mobile Phase A | % Mobile Phase B |
|---|---|---|
| 0 | 60 | 40 |
| 3.0 | 50 | 50 |
| 14.0 | 39 | 61 |
| 14.5 | 60 | 40 |

Retention time of embodiments of the present invention is preferably within about 8 to about 12 minutes.

Standard Preparation: Accurately weigh about 20 mg of the pharmaceutical composition into a 100 mL volumetric flask. Vortex to dissolve in, and dilute to volume with Diluent.

Sample Preparation: Carefully remove tablets from not less than 10 capsules and clean away any placebo powder by blowing gently with air. Accurately record the total tablet weight and grind them into a fine powder. Transfer an accurately weighed portion of the powder, equivalent to about 200 mg of the pharmaceutical composition, into a 100 mL volumetric flask. Add Diluent to about half of the flask and shake for about 30 minutes on mechanical shaker. Dilute to volume with Diluent, mix well and filter a portion through a 0.45 μm membrane filter (Millex-HV, or equivalent). Further dilute 5.0 mL to 50 .0 mL with Diluent.

Placebo Preparation: Accurately weigh about 150 mg of placebo powder into a 100 mL volumetric flask. Add Diluent to about half of the flask and shake for about 30 minutes on mechanical shaker. Dilute to volume with Diluent, mix well and filter a portion through a 0.45 μm membrane filter (Millex-HV, or equivalent). Further dilute 5.0 mL to 50.0 mL with Diluent.

System Suitability (See General Chapter Chromatography <621> of the U.S. Pharmacopeia): Chromatograph the Standard preparation and record the peak responses as directed under Procedure. The relative standard deviation

of embodiments of the present invention's peak areas for replicate injections is preferably NMT about 5.0%, more preferably NMT about 2.0%. The tailing factor of embodiments of the present invention is preferably NMT about 5.0, more preferably NMT about 2.0.

Procedure: Separately inject equal volumes (about 10 μL) of Diluent, Placebo, Standard and Sample preparations into the chromatograph, record the chromatograms, and measure the responses for the major peaks.

Calculate the assay values using the following formula:

$$\%Assay = \frac{R_u}{R_s} \times \frac{StdWt\,(mg)}{Std\ Dil\,(mL)} \times P \times \frac{100\,(mL)}{SplWt(mg)} \times 10 \times \frac{ATW\ (mg)\ x\,100}{LC(mg\,/\,cap)}$$

where:

$R_u$ = Formulation peak area obtained from the sample preparation
$R_s$ = Formulation peak area obtained from the standard preparation
P = Purity of Reference Standard
Std Wt = Standard weight (mg)
Std Dil = Standard dilution (mL)
Spl Wt = Sample weight (mg)
ATW = Averaged Tablet Weight
LC = Label claim (mg/cap)

Disregarding peaks originated from Diluent and Placebo, calculate the percentage w/w of individual and total impurities by the formulas:

$$\%\,individual\ impurity = \frac{R_i}{R_u} \times\ 100$$

*Total impurities (% w/w) = ∑%(w/w) individual impurity*
where:

$R_i$ = Impurity peak area obtained from the Sample preparation.
$R_u$ = Formulation peak area obtained from the Sample preparation.

Embodiments of the present invention include pharmaceutical compositions of difimicin, including different polymorph forms and derivatives thereof, and combinations thereof. Therapeutically effective dosage amounts of difimicin, generally range from about 1 mg to about 1000 mg, preferably from about 5 mg to about 500 mg, and more preferably from about 25 mg to about 500 mg. Exemplary dosages therefore include, but are not limited to, about 25 mg, about 50 mg, about 75 mg, about 100 mg, about 125 mg, about 150 mg, about 175 mg, about 200 mg, about 300 mg, about 450 mg, and about 500 mg, preferably about 50 mg, about 100 mg, and about 200 mg.

In some embodiments, difimicin is administered with one or more of related compound A (RRT = 0.71, 1028 mass), related compound B (RRT = 0.75, 989 mass), related compound C (RRT = 0.78, 0.81 mass), related compound D (RRT = 0.81, 970 mass), related compound E (RRT = 0.84, 1042 mass), related compound F (RRT = 0.86, 1022 mass), related compound G (RRT = 0.88, 1042 mass), related compound H (RRT = 0.98, 1042 mass), related compound I (RRT = 1.03, 1040 mass), related compound J (RRT = 1.07, 1056 mass), related compound K (RRT = 1.11, 1040 mass), related compound L (RRT = 1.13, 1070 mass), related compound M (RRT = 1.13, 1054 mass), related compound N (RRT = 1.19, 1070 mass), related compound O (RRT = 1.23, 1054 mass), lipiarmycin A4 (RRT = 0.89, 1042 mass), tiacumicin C (RRT = 0.95, 1056 mass), and tiacumicin F (RRT = 0.92, 1056 mass). Optionally, other tiacumicins, such as tiacumicin A (RRT 1.10) may also be included in embodiments of the present invention. Figure 1 discloses the general structures of these compounds. In some embodiments, the pharmaceutical compositions contains less than about 20%, preferably less than or equal to about 10% of such substances, such as about 5%. For example, some embodiments contain at time 0: less than about 10% of related compounds A to O, preferably less than or equal to about 5%, such as about 1%; less than about 10% of lipiarmycin A4, preferably less than or equal to about 5%, such as about 1.5%; less than about 10% of Tiacumicin A, Tiacumicin C, and/or Tiacumicin F, preferably less than or equal to about 5%, such as about 1%.

Some embodiments of the present invention may be characterized at time 0 by the HPLC profile substantially depicted by the chromatogram of Figure 4, or as a stressed tablet after two months at 40°C/75%RH by the HPLC profile

substantially depicted by the chromatogram of Figure 5.

Antioxidants are one or more of the following: butylated hydroxyanisole (BHA), butylated hydroxytoluene (BHT), ascorbic acid, ascorbyl palmitate, propyl gallate, dodecyl gallate, ethyl gallate, octyl gallate, alpha tocopherol, sodium ascorbate, sodium metabisulfite, fumaric acid, malic acid, preferably butylated hydroxytoluene (BHT). A stabilizing amount of one or more antioxidants are generally from about 0.001% to about 50% of the total weight of the composition, preferably from about 0.01% to about 25% of the total weight of the composition. For example, in some embodiments of the present invention, a stabilizing amount of butylated hydroxytoluene (BHT) can be from about 0.001% to about 5% of the total weight of the composition, preferably from about 0.01% to about 0.5% of the total weight of the composition, and more preferably from about 0.01% to about 0.15% of the total weight of the composition.

The one or more antioxidants, such as BHT, may be added to embodiments of the present invention as a dry powder, in a solution (for example, using solvents such as, but not limited to, isopropyl alchohol and methanol), or by any other forms known to those of ordinary skill in the art.

Pharmaceutical compositions of the present invention may be used for the treatment or prevention of a disease, infection, and/or other condition associated with the use of antibiotics, cancer chemotherapies, or antiviral therapies. The diseases, infections, and/or other conditions may include, but are not limited to, the following: C. *difficile*-associated diarrhea (CDAD), colitis, pseudomembranous colitis, antibiotic associated diarrhea and infections due to C. *difficile,* C. *perfringens, Staphylococcus* species, or *Enterococcus,* clostridial enterocolitis, neonatal diarrhea, antibiotic-associated enterocolitis, sporadic enterocolitis, nosocomial enterocolitis, colitis membranous, infectious diarrhea, and irritable bowel syndrome. In a preferred embodiment, the disease, infection, and/or other condition is C. *difficile*-associated diarrhea (CDAD).

Pharmaceutical compositions of embodiments of the present invention may be prepared for administration orally, rectally, vaginally, transmucosally, transdermally, parenterally, subcutaneously, intramuscularly, or intravenously, preferably orally. The compositions can be administered daily (e.g., once, twice, three times, or four times daily) or less frequently (e.g., once every other day, or one or twice weekly). For example, in some embodiments, difimicin can be administered in an amount of about 50 mg to about 200 mg once or twice daily.

The compositions of the present invention may further comprise one or more pharmaceutically acceptable excipients or inactive ingredients, which are suitable for these methods of administration and are generally known to those of skill in the art. Inactive ingredients, for example, may solubilize, suspend, thicken, dilute, lubricate, emulsify, further stabilize, preserve, protect, color, flavor, and/or fashion the active ingredients into an applicable and efficacious preparation that is safe, convenient, and otherwise acceptable for use. Further, excipients can be included according to the judgment of the pharmaceutical scientist formulating the medicament. In addition, other active ingredients can be included to produce a dual- or multiple-ingredient medication.

For example, one or more inert diluents and/or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); one or more granulating and disintegrating agents (e.g., cellulose derivatives including, but not limited to, microcrystalline cellulose, starches including potato starch, croscarmellose sodium, alginates, or alginic acid); one or more binding agents (e.g., sucrose, glucose, mannitol, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropylmethylcellulose, ethylcellulose, polyvinylpyrrolidone, or polyethylene glycol); and one or more lubricating agents, glidants, and antiadhesives (e.g., magnesium stearate, zinc stearate, stearic acid, silicas, hydrogenated vegetable oils, or talc), and combinations thereof. Other pharmaceutically acceptable excipients can be colorants, flavoring agents, plasticizers, humectants, buffering agents, and the like, which are found, for example, in The Handbook of Pharmaceutical Excipients, third edition, edited by Authur H. Kibbe, American Pharmaceutical Association, Washington, DC.

Solid dosage forms that can be prepared from the pharmaceutical compositions of embodiments of the present invention can include tablets, caplets, capsules, rectal or vaginal suppositories, pills, dragees, lozenges, granules, beads, microspheres, pellets, and powders, or any combination thereof. Formulations also can be prepared in the form of solutions, suspensions, emulsions, syrups, and elixirs. These liquid dosage forms can include liquid diluents in addition to the solid ingredients discussed above. Such diluents can include, but are not limited to solvents, solubilizing agents, suspending agents and emulsifiers such as water or saline solutions, ethanol and other pharmaceutically acceptable alcohols, ethyl carbonate, ethyl acetate, propylene glycol, dimethyl formamide, pharmaceutically acceptable oils such as cottonseed, corn, olive, castor and sesame, fatty acid esters of sorbitan, polyoxyethylene sorbitol, and agar-agar. Acid and neutral diluents are generally preferred, and more preferably acid diluents.

The pharmaceutical composition of embodiments of the present invention can be used for any convenient dosage amount of the active ingredient. Generally, the level of the active ingredient can be increased or decreased according to the judgment of the physician, pharmacist, pharmaceutical scientist or other person of skill in the art. The amount of the remaining non-active ingredients can be adjusted as needed.

Embodiments of the present invention can be either immediate or modified release (e.g. pharmaceutical compositions that create a substantially constant concentration of the drug within the intestinal tract over an extended period of time, and pharmaceutical compositions that have modified release characteristics based on temporal or environmental criteria. See, for example, Modified-Release Drug Delivery Technology, eds. M. J. Rathbone, J. Hodgraft and M.S. Roberts. Marcel Dekker, Inc. New York).

For example, in some embodiments of the present invention, an immediate release tablet comprises one or more pharmaceutically acceptable excipients including, but not limited to, one or more of microcrystalline cellulose, starch, hydroxypropylcellulose, lactose monohydrate, anhydrous lactose, talc, colloidal silicon dioxide, providone, citric acid, poloxamer, sodium starch glycolate, stearic acid, and magnesium stearate. In one embodiment, the one or more pharmaceutically acceptable excipients include, but are not limited to, one or more of microcrystalline cellulose, starch, hydroxypropylcellulose, sodium starch glycolate, and magnesium stearate. Microcrystalline cellulose can be present from about 1% to about 90% of the total weight of the composition, preferably from about 5% to about 50% of the total weight of the composition. Starch can be present from about 1% to about 25% of the total weight of the composition. Hydroxpropylcellulose can be present from about 0.01% to about 25% of the total weight of the composition, preferably from about 0.05% to about 10% of the total weight of the composition. Sodium starch glycolate can be present from about 0.01% to about 25% of the total weight of the composition, preferably from about 0.05% to about 10% of the total weight of the composition. Magnesium stearate can be present from about 0.01% to about 25% of the total weight of the composition, preferably from about 0.05% to about 10% of the total weight of the composition.

Some embodiments of the present invention can include one or more coatings. The coating (s) can be applied by any conventional technique such as pan coating, fluid bed coating or spray coating. The coating(s) can be applied as a suspension, spray, dust, or powder. The coating(s) can be formulated for immediate release, delayed/enteric release or sustained release of the second pharmaceutical active in accordance with methods well known in the art. Conventional coating techniques are described, for example in Remington's Pharmaceutical Sciences, 18th Ed. (1990).

An immediate release coating is commonly used to improve product elegance as well as for a moisture barrier, and taste and odor masking. Rapid breakdown of the film in gastric media is important, leading to effective disintegration and dissolution. EUDRAGIT® RD100 (Rohm) is an example of such a coating. It is a combination of a water insoluble cationic methacrylate copolymer with a water soluble cellulose ether. In powder form, it is readily dispensable into an easily sprayable suspension that dries to leave a smooth film. Such films rapidly disintegrate in aqueous media at a rate that is independent of pH and film thickness.

A protective coating layer (i.e., seal coat) can be applied, if desired, by conventional coating techniques such as pan coating or fluid bed coating using solutions of polymers in water or suitable organic solvents or by using aqueous polymer dispersions. Suitable materials for the protective layer include cellulose derivatives such as hydroxyethyl cellulose, hydroxypropyl cellulose, hydroxypropyl methylcellulose, polyvinylpyrrolidone, polyvinylpyrrolidone/vinyl acetate copolymer, ethyl cellulose aqueous dispersions, and the like. The protective coating layer can include one or more additional antioxidants, chelating agents, colors, or dyes.

An enteric coating layer can be applied onto the cores with or without seal coating by conventional coating techniques, such as pan coating or fluid bed coating using solutions of polymers in water or suitable organic solvents or by using aqueous polymer dispersions. All commercially available pH-sensitive polymers are included. The pharmaceutical active is not released in the acidic stomach environment of approximately below pH 4.5, but not limited to this value. The pharmaceutical active should become available when the pH-sensitive layer dissolves at the greater pH, after a certain delayed time, or after the unit passes through the stomach. The preferred delay time is in the range of one to six hours.

Enteric polymers include, but are not limited to, cellulose acetate phthalate, cellulose acetate trimellitate, hydroxypropyl methylcellulose phthalate, polyvinyl acetate phthalate, carboxymethylethylcellulose, co-polymerized methacrylic acid/methacrylic acid methyl esters such as, for instance, materials known under the trade name EUDRAGIT L12.5, L100, or EUDRAGIT S12.5, S100 or similar compounds used to obtain enteric coatings. Aqueous colloidal polymer dispersions or re-dispersions can be also applied, e.g. EUDRAGIT L 30D-55, EUDRAGIT L100-55, EUDRAGIT S100, EUDRAGIT preparation 4110D (Rohm Pharma); AQUATERIC, AQUACOAT CPD 30 (FMC); KOLLICOAT MAE 30D and 30DP (BASF); EASTACRYL 30D (Eastman Chemical).

A sustained release film coat can include a water insoluble material such as a wax or a wax-like substance, fatty alcohols, shellac, zein, hydrogenated vegetable oils, water insoluble celluloses, polymers of acrylic and/or methacrylic acid, and any other slowly digestible or dispersible solids known in the art. The solvent for the hydrophobic coating material can be organic or aqueous. Preferably, the hydrophobic polymer is selected from (i) a water insoluble cellulosic polymer, such as an alkylcellulose, preferably ethylcellulose; (ii) an acrylic polymer; or (iii) mixtures thereof. In other preferred embodiments of the present invention, the hydrophobic material comprising the controlled release coating is an acrylic polymer. Any acrylic polymer which is pharmaceutically acceptable can be used for the purposes of the present invention. The acrylic polymers can be cationic, anionic or nonionic polymers and can be acrylates, methacrylates, formed of methacrylic acid, or methacrylic acid esters. Examples of suitable acrylic polymers include but are not limited to acrylic acid and methacrylic acid copolymers, methacrylic acid copolymers, methyl methacrylate copolymers, ethoxyethyl methacrylates, cyanoethyl methacrylate, methyl methacrylate, copolymers, methacrylic acid copolymers, methyl methacrylate copolymers, methyl methacrylate copolymers, methyl methacrylate copolymers, methacrylic acid copolymer, aminoalkyl methacrylate copolymer, methacrylic acid copolymers, methyl methacrylate copolymers, poly(acrylic acid), poly(methacrylic acid, methacrylic acid alkylamine copolymer, poly(methyl methacrylate), poly(methacrylic acid) (anhydride), methyl methacrylate, polymethacrylate, methyl methacrylate copolymer, poly(methyl methacrylate), poly(methyl methacrylate) copolymer, polyacrylamide, aminoalkyl methacrylate copolymer, poly(methacrylic acid anhydride), and glycidyl methacrylate copolymers.

A barrier coat can be included between a coating and another coating or the exterior of the preliminary dosage form (e.g., the compressed tablet, the capsule shell, etc.). The barrier coat can be comprised of an enteric/delayed release coat (as above), or a barrier (non-functional) layer, which serves as a protective coat to prevent moisture from contacting the inner pharmaceutical component, or to prevent leaching from inside the barrier coat to an outer pharmaceutically active component or vice versa. A moisture barrier coat may be comprised of any applicable type of coat known to those of skill in the art.

In some embodiments of the present invention, the solid ingredients of the formulation are blended, optionally granulated, such as by dry or wet granulation, and compressed into tablets, and optionally coated. Compression and/or coating can be accomplished by standard industry means. If applicable, the pan speed and the target spray rate can be adjusted to suit the particular tablet being coated. Any suitable coating can be used in accordance with the present invention.

In other embodiments, the pharmaceutical composition can be used to fill capsules such as hard gelatin capsules or used to prepare any other convenient solid dosage form. Compositions according to the invention can be stored in the form of powders, granulates, intermediates, suspensions, or solutions prior to addition of additional desired pharmaceutical excipients for the production of final dosage forms such as tablets or solid-filled capsules, or final liquid dosage forms such as solutions, syrups, suspensions, emulsions, and the like.

The solid dosage forms of the embodiments of the present invention can be of any color or combination of one or more colors. The solid dosage forms can also be of any shape, for example, flat and/or oval-shaped.

The solid dosage forms can be dispensed in any form. For example, tablets or capsules can be dispensed in blister packs (e.g., ACLAR® 2000 or PVDC blister packs, preferably aluminum-aluminum blister packs) or high-density polyethylene (HDPE) bottles, which preferably include a desiccant and/or an induction seal, such as a child-resistant closure with an induction seal. Any number of tablets or capsules may be included in a unit dose package, such as a blister pack, including but not limited to 2, 4, 6, 8, 10, 12, 16, 20, 24, 48, 56, 75 or 100 tablets or capsules.

The following examples further illustrate the present invention and are not to be construed to limit the present invention in any manner.

EXAMPLE 1: Pharmaceutical Composition of Difimicin

A pharmaceutical composition of difimicin was prepared with the ingredients shown in Table I.

Table I

| Ingredient | Weight/tablet (mg) |
|---|---|
| Difimicin | 200.0 |
| Microcrystalline cellulose | 76.7 |
| Starch | 40.0 |
| Hydroxypropylcellulose | 16 |
| Butylated hydroxytoluene (BHT) | 0.3 |
| Sodium Starch Glycolate | 6.0 |
| Methanol | (not in finished product) |
| Purified water | (not in finished product) |
| Magnesium stearate | 3 |

Difimicin was mixed with microcrystalline cellulose (e.g., Avicel PH 101), starch (e.g., Starch 1500), sodium starch glycolate, and hydroxypropylcellulose. The mixture was sprayed with a solution of BHT in methanol. The sprayed mixture was granulated with hydroxypropylcellulose by high shear granulation in water, and dried in a fluid bed dryer. More sodium starch glycolate was added. The resultant composition was lubricated with magnesium stearate and

compressed into a capsule-shaped, biconvex tablet. Some of the tablets were used with microcrystalline cellulose powder to fill grey coni-snap capsules, size 0, prior to compression. No less than 85% of the active ingredients in the solid dosage forms dissolved in 30 minutes in 900 mL of a 3.0% medium of sodium lauryl sulphate by the USP paddle method at 100 rpm and 37°C.

EXAMPLE 2: A Comparison of the Stability of Formulations of Difimicin

The stability of the formulations of Table II, having difimicin with BHT, BHA, or no anti-oxidant were compared in Table III. The tablets were stored at 40°C at 75% relative humidity (RH) in standard HDPE pharmaceutical containers with inductions seals, and with or without a desiccant. Samples of these tablets were analyzed for impurity levels using a high-performance liquid chromatography (HPLC) standard assay.

Table II

| Ingredients | Formulation (no antioxidant) (mg) | Formulation with antioxidant (mg) |
|---|---|---|
| Difimicin | 200 | 200 |
| Microcrystalline cellulose | 83 | 83 |
| Starch | 40 | 40 |
| Sodium Starch Glycolate | 6 | 6 |
| Hydroxypropylcellulose | 16 | 16 |
| BHT or BHA | N/A | 0.3 |
| Isopropyl alcohol | N/A | (not in finished |
| | | product) |
| Sodium Starch Glycolate | 8 | 8 |
| Purified water | (not in finished product) | (not in finished product) |
| Magnesium stearate | 3 | 3 |

Table III

| | Time | Assay % | % Related Compound L (RRT 1.13) |
|---|---|---|---|
| Difimicin (no antioxidant) | Initial | 102.9 | 0.314 |
| | 1 month (with desiccant) | 95.4 | 0.398 |
| | 2 months (with desiccant) | 97.3 | 0.450 |
| | 2 months (no desiccant) | 99.7 | 0.460 |
| Difimicin with BHA | Initial | 99.4 | 0.306 |
| | 1 month (with desiccant) | 96.0 | 0.381 |
| | 2 months (with desiccant) | 97.1 | 0.389 |
| | 2 months (no desiccant) | 97.1 | 0.368 |
| Difimicin with BHT | Initial | 100.2 | 0.312 |
| | 1 month (with desiccant) | 96.5 | 0.338 |
| | 2 months (with desiccant) | 97.8 | 0.370 |
| | 2 months (no desiccant) | 98.1 | 0.350 |

Related Compound L in Table III, having a retention time relative (RRT) of 1.13, is believed to be an oxidation product of difimicin. FIG. 1 discloses a possible structure of related Compound L.

EXAMPLE 3: Stability with different dosage forms and packaging

As shown in Table IV, the stability of pharmaceutical compositions of the present invention with difimicin with different solid dosage forms and packaging was compared at 25°C/60% RH.

Table IV

| Conditions | Time | Dissolution (% in 30 minutes) | Assay (%) |
|---|---|---|---|
| HDPE Bottles | | | |
| core tablets, induction seal, without desiccant | Initial | 102 | 99.0 |
| | 1 month* | -- | -- |
| | 2 months | 97 | 97.0 |
| | 3 months | 93 | 95.8 |
| core tablets, induction seal, with desiccant | Initial | 102 | 99.0 |
| | 1 month* | -- | -- |
| | 2 months | 98 | 96.5 |
| | 3 months | 95 | 98.1 |
| over-encapsulated, no seal, with desiccant | Initial | 102 | 99.0 |
| | 1 month | 97 | 99.4 |
| | 2 months* | -- | -- |
| | 3 months | 99 | 98.8 |
| over-encapsulated, induction | Initial | 102 | 99.0 |
| | 1 month | -- | 99.2 |
| seal, with desiccant | 2 months | 98 | 98.2 |
| | 3 months | 99 | 97.3 |
| Blister Packs | | | |
| core tablets, Al-Al blister packs | Initial | 98 | 100.6 |
| | 1 month | 97 | 100.2 |
| | 2 months* | -- | -- |
| | 3 months* | -- | -- |
| * Data not collected at this time period. | | | |

## Claims

1. A pharmaceutical composition comprising a therapeutically effective amount of a compound with the formula:

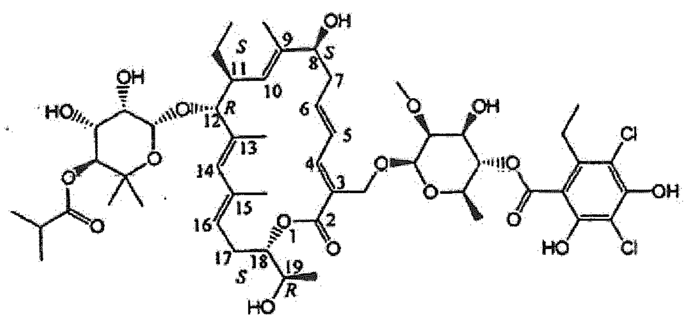

a stabilizing amount of one or more antioxidants selected from the group consisting of butylated hydroxyanisole, butylated hydroxytoluene, ascorbic acid, ascorbyl palmitate, propyl gallate, dodecyl gallate, ethyl gallate, octyl gallate, alpha tocopherol, sodium ascorbate, sodium metabisulfite, fumaric acid, and malic acid, and optionally one or more pharmaceutical acceptable excipients selected from the group consisting of microcrystalline cellulose, starch, hydroxypropylcellulose, sodium starch glycolate, isopropyl alcohol, magnesium stearate, and combinations thereof.

**2.** The composition of claim 1, wherein the pharmaceutical composition is substantially stable in the presence of humidity.

**3.** The composition of claim 1, wherein the pharmaceutical composition is substantially stable in the presence of heat.

**4.** The composition of claim 1, wherein the stabilizing amount of one or more antioxidants is from about 0.001% to about 50% of the total weight of said composition.

**5.** The composition of claim 1, wherein the one or more antioxidants is butylated hydroxytoluene.

**6.** The composition of claim 1, wherein the composition is administered orally.

**7.** The composition of claim 1, wherein the composition is in a solid dosage form.

**8.** The composition of claim 7, wherein the solid dosage form is a tablet.

**9.** The composition of claim 7, wherein the solid dosage form is dispensed in a unit dose package.

**10.** The pharmaceutical composition of claim 1 for use in treating or preventing a disease, infection, and/or other condition associated with the use of antibiotics, cancer chemotherapies, or antiviral therapies in a subject.

**11.** The pharmaceutical composition for use according to claim 10, wherein the disease, infection, and/or other condition is selected from the following: C. *difficile*-associated diarrhea (CDAD), colitis, pseudomembranous colitis, antibiotic associated diarrhea and infections due to C. *difficile*, C. *perfringens, Staphylococcus* species, or *Enterococcus,* clostridial enterocolitis, neonatal diarrhea, antibiotic-associated enterocolitis, sporadic enterocolitis, nosocomial enterocolitis, colitis membranous, infectious diarrhea, and irritable bowel syndrome.

**12.** The pharmaceutical composition for use according to claim 11, wherein the disease, infection, and/or other condition is C. *difficile*-associated diarrhea (CDAD).

**13.** The pharmaceutical composition of claim 1, wherein the composition comprises a therapeutically effective amount of the compound with the formula :

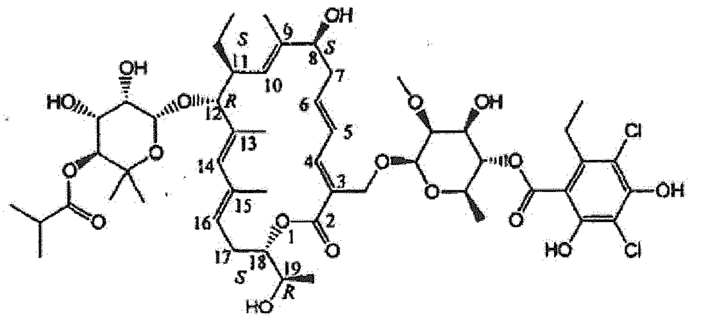

butylated hydroxytoluene in an amount of about 0.001% to about 5% of the total weight of said composition, and optionally one or more of microcrystalline cellulose, starch, hydroxypropylcellulose, sodium starch glycolate, and magnesium stearate.

**Patentansprüche**

1. Eine pharmazeutische Zusammensetzung, umfassend eine therapeutisch wirksame Menge einer Verbindung der Formel:

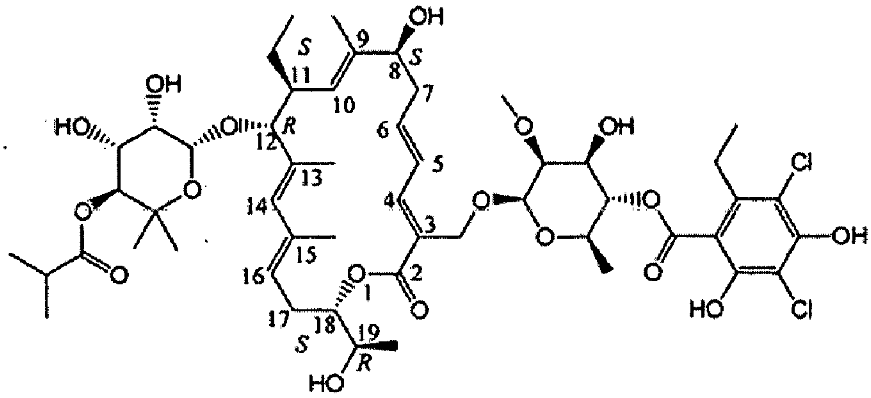

,

eine stabilisierende Menge eines oder mehrerer Antioxidanzien, ausgewählt aus der Gruppe, bestehend aus butyliertem Hydroxyanisol, butyliertem Hydroxytoluol, Ascorbinsäure, Ascorbylpalmitat, Propylgallat, Dodecylgallat, Ethylgallat, Octylgallat, alpha-Tocopherol, Natriumascorbat, Natriummetabisulfit, Fumarsäure und Äpfelsäure, und gegebenenfalls einen oder mehrere pharmazeutisch annehmbare Hilfsstoffe, ausgewählt aus der Gruppe, bestehend aus mikrokristalliner Cellulose, Stärke, Hydroxypropylcellulose, Natriumstärkeglykolat, Isopropylalkohol, Magnesiumstearat und Kombinationen davon.

2. Die Zusammensetzung nach Anspruch 1, wobei die pharmazeutische Zusammensetzung in Gegenwart von Feuchtigkeit im Wesentlichen stabil ist.

3. Die Zusammensetzung nach Anspruch 1, wobei die pharmazeutische Zusammensetzung in Gegenwart von Wärme im Wesentlichen stabil ist.

4. Die Zusammensetzung nach Anspruch 1, wobei die stabilisierende Menge des einen oder der mehreren Antioxidanzien etwa 0,001% bis etwa 50% des Gesamtgewichts der Zusammensetzung ausmacht.

5. Die Zusammensetzung nach Anspruch 1, wobei das eine oder die mehreren Antioxidanzien butyliertes Hydroxytoluol ist.

6. Die Zusammensetzung nach Anspruch 1, wobei die Zusammensetzung oral verabreicht wird.

7. Die Zusammensetzung nach Anspruch 1, wobei die Zusammensetzung in einer festen Darreichungsform vorliegt.

8. Die Zusammensetzung nach Anspruch 7, wobei die feste Darreichungsform eine Tablette ist.

9. Die Zusammensetzung nach Anspruch 7, wobei die feste Darreichungsform in einer Unit-Dose-Packung verabreicht wird.

10. Die pharmazeutische Zusammensetzung nach Anspruch 1 zur Verwendung bei der Behandlung oder Prävention einer Erkrankung, Infektion und/oder eines anderen Zustands, der mit der Verwendung von Antibiotika, Krebs-Chemotherapien oder Antivirus-Therapien in Zusammenhang steht, bei einem Subjekt.

11. Die pharmazeutische Zusammensetzung zur Verwendung gemäß Anspruch 10, wobei die Erkrankung, Infektion und/oder der andere Zustand ausgewählt ist aus den folgenden: *C.-difficile*-assoziierte Diarrhö (CDAD), Colitis, pseudomembranöse Colitis, Antibiotikum-assoziierte Diarrhö und durch C. *difficile, C. perfringens, Staphylococcus*- oder *Enterococcus-Arten* hervorgerufene Infektionen, clostridiale Enterocolitis, neonatale Diarrhö, Antibiotikum-assoziierte Enterocolitis, sporadische Enterocolitis, nosokomiale Enterocolitis, Colitis membranous, infektiöse Diarrhö und Reizdarmsyndrom.

12. Die pharmazeutische Zusammensetzung zur Verwendung gemäß Anspruch 11, wobei die Erkrankung, Infektion und/oder der andere Zustand *C.-difficile*-assoziierte Diarrhö (CDAD) ist.

13. Die pharmazeutische Zusammensetzung nach Anspruch 1, wobei die Zusammensetzung eine therapeutisch wirksame Menge der Verbindung der Formel:

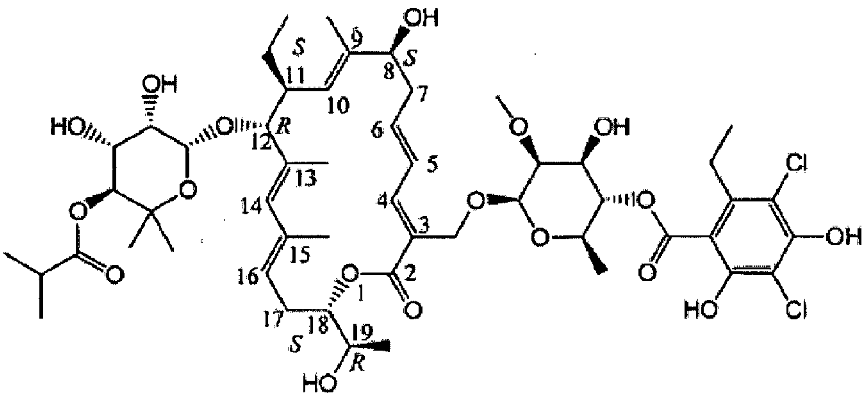

,

butyliertes Hydroxytoluol in einer Menge von etwa 0,001% bis etwa 5% des Gesamtgewichts der Zusammensetzung, und gegebenenfalls eines oder mehrere von mikrokristalliner Cellulose, Stärke, Hydroxypropylcellulose, Natriumstärkeglykolat und Magnesiumstearat umfasst.

## Revendications

1. Composition pharmaceutique comprenant une quantité thérapeutiquement efficace d'un composé ayant la formule:

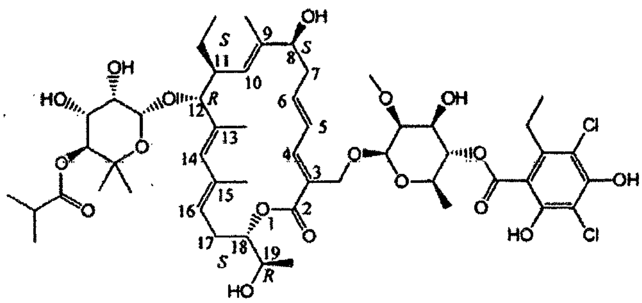

une quantité stabilisante d'un ou plusieurs antioxydants choisis dans le groupe constitué par l'hydroxyanisole butylé, l'hydroxytoluène butylé, l'acide ascorbique, le palmitate d'ascorbyle, le gallate de propyle, le gallate de dodécyle, le gallate d'éthyle, le gallate d'octyle, l'alpha tocophérol, l'ascorbate de sodium, le métabisulfite de sodium, l'acide fumarique et l'acide malique, et facultativement un ou plusieurs excipients pharmaceutiquement acceptables choisis dans le groupe constitué par une cellulose microcristalline, un amidon, une hydroxypropylcellulose, un glycolate d'amidon sodique, un alcool isopropylique, un stéarate de magnésium et des combinaisons de ceux-ci.

2. Composition selon la revendication 1, dans laquelle la composition pharmaceutique est essentiellement stable en présence d'humidité.

3. Composition selon la revendication 1, dans laquelle la composition pharmaceutique est essentiellement stable en présence de chaleur.

4. Composition selon la revendication 1, dans laquelle la quantité stabilisante d'un ou plusieurs antioxydants est d'environ 0,001% à environ 50% du poids total de ladite composition.

5. Composition selon la revendication 1, dans laquelle le ou les antioxydant(s) est (sont) de l'hydroxytoluène butylé.

**6.** Composition selon la revendication 1, dans laquelle la composition est administrée oralement.

**7.** Composition selon la revendication 1, dans laquelle la composition est sous une forme de dosage solide.

**8.** Composition selon la revendication 7, dans laquelle la forme de dosage solide est un comprimé.

**9.** Composition selon la revendication 7, dans laquelle la forme de dosage solide est présentée dans un paquet de doses unitaires.

**10.** Composition pharmaceutique selon la revendication 1, pour l'utilisation dans le traitement ou la prévention d'une maladie, d'une infection et/ou d'une autre affection associée à l'utilisation d'antibiotiques, de chimiothérapies anticancéreuses ou de thérapies antivirales chez un sujet.

**11.** Composition pharmaceutique pour l'utilisation selon la revendication 10, dans laquelle la maladie, l'infection et/ou l'autre affection est choisie parmi les suivantes: diarrhée associée à C. *difficile* (CDAD), colite, colite pseudomembraneuse, diarrhée associée à un antibiotique et infections dues à C. *difficile, C. perfringens,* les espèces *Staphylococcus,* ou *Enterococcus*, entérocolite à clostridium, diarrhée néonatale, entérocolite associée à un antibiotique, entérocolite sporadique, entérocolite nosocomiale, colite membraneuse, diarrhée infectieuse et syndrome du côlon irritable.

**12.** Composition pharmaceutique pour l'utilisation selon la revendication 11, dans laquelle la maladie, l'infection et/ou l'autre affection est une diarrhée associée à C. *difficile* (CDAD).

**13.** Composition pharmaceutique selon la revendication 1, dans laquelle la composition comprend une quantité thérapeutiquement efficace du composé ayant la formule:

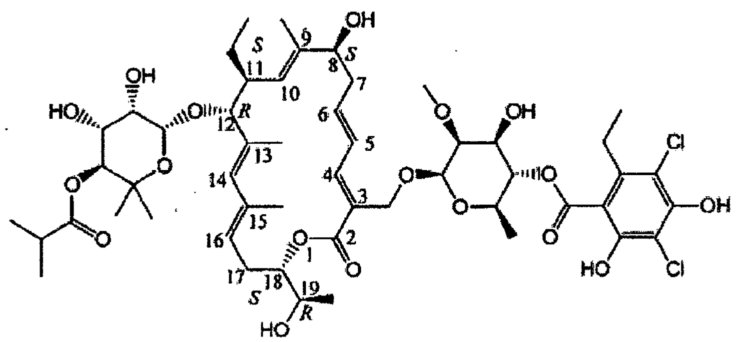

de l'hydroxytoluène butylé en une quantité d'environ 0,001% à environ 5% du poids total de ladite composition, et facultativement un ou plusieurs ingrédients parmi une cellulose microcristalline, un amidon, une hydroxypropylcellulose, un glycolate d'amidon sodique et un stéarate de magnésium.

FIGURE 1.

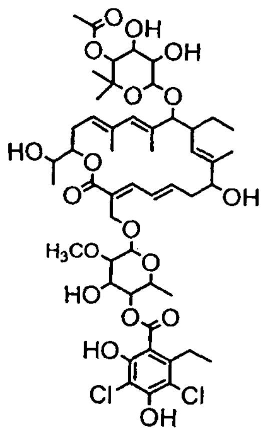

Related Compound A

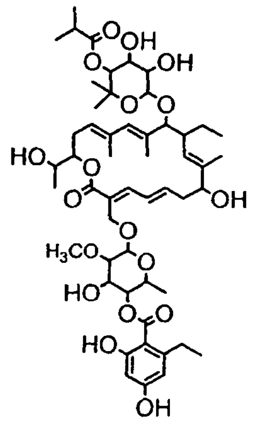

Related Compound B

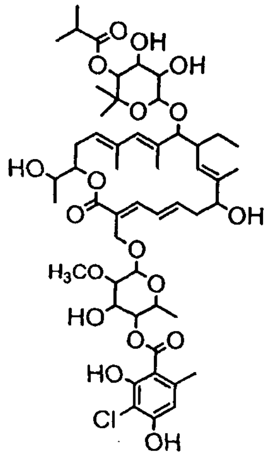

Related Compound C

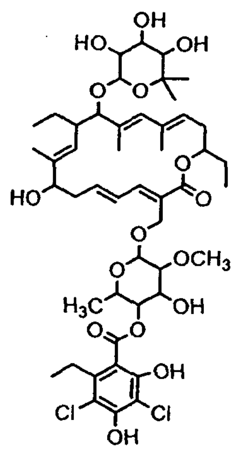

Related Compound D

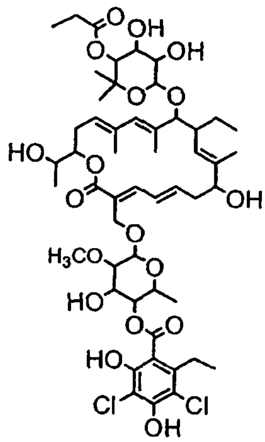

Related Compound E

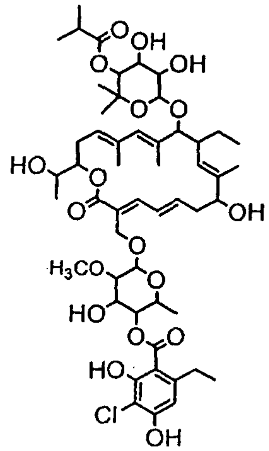

Related Compound F

FIG. 1 (cont.)

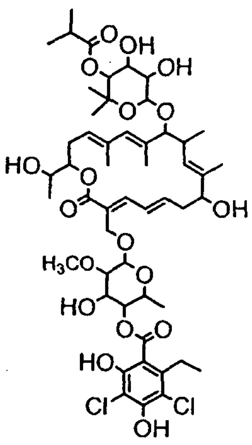

Related Compound G

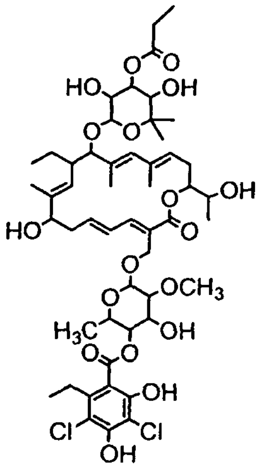

Related Compound H

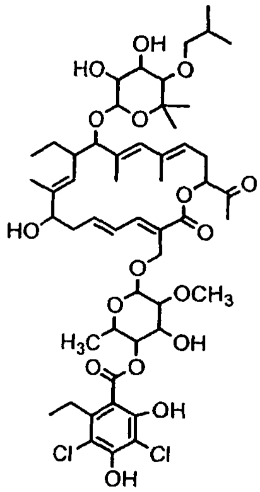

Related Compound I

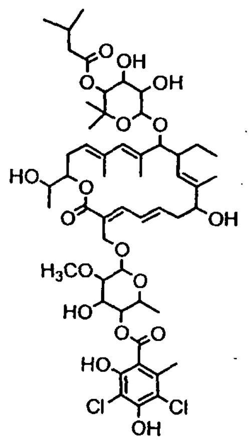

Related Compound J

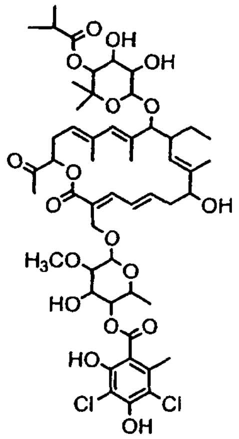

Related Compound K

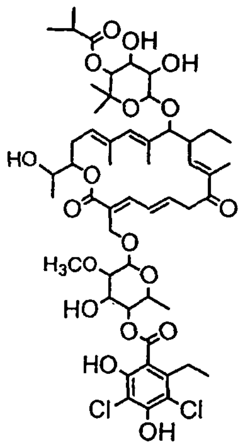

Related Compound L

FIG. 1 (cont.)

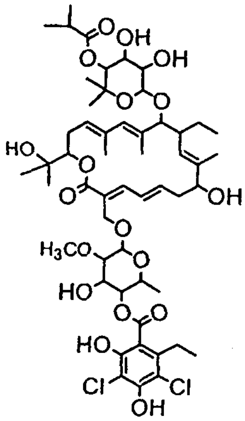

Related Compound M

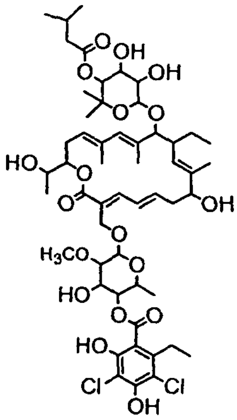

Related Compound N

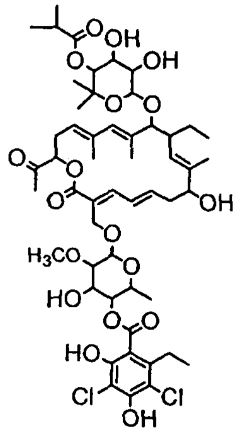

Related Compound O

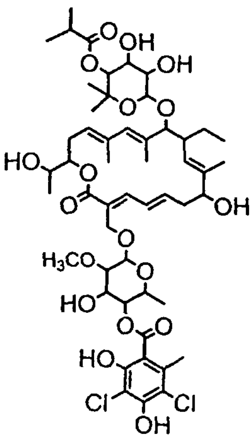

Lipiarmycin A4

FIG. 1 (cont.)

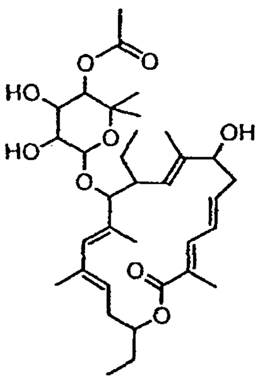

Tiacumicin A

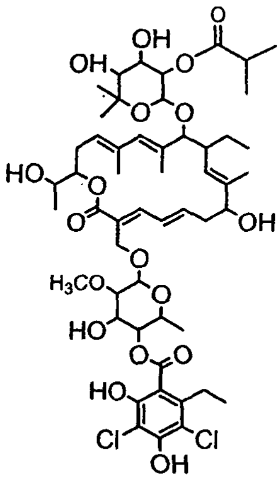

Tiacumicin C

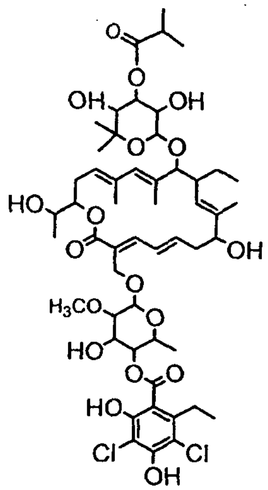

Tiacumicin F

FIGURE 2

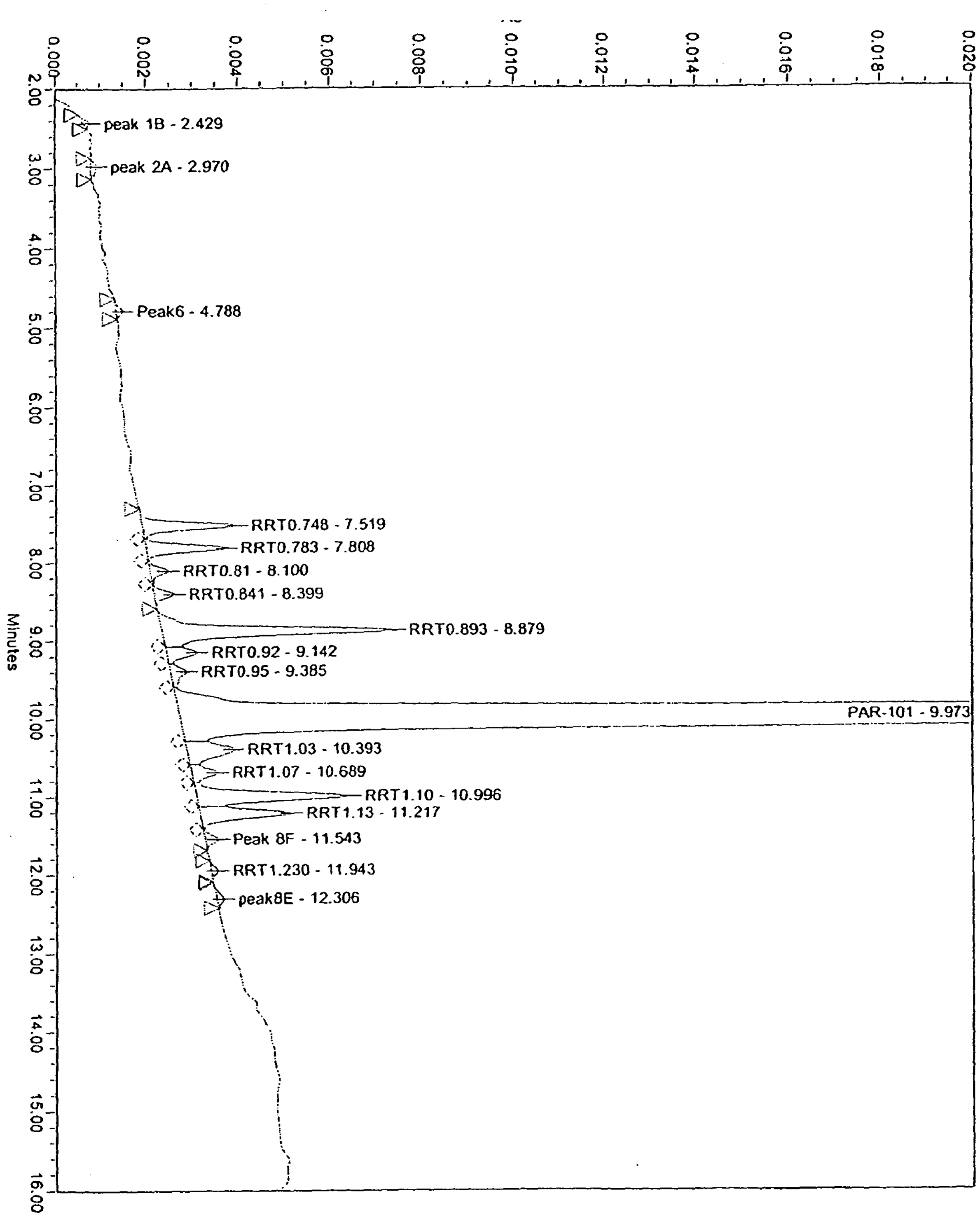

0.000
0.002
0.004
0.006
0.008
0.010
0.012
0.014
0.016
0.018
0.020
Minutes
peak 1B - 2.429
peak 2A - 2.970
Peak6 - 4.788
RRT0.748 - 7.519
RRT0.783 - 7.808
RRT0.81 - 8.100
RRT0.841 - 8.399
RRT0.893 - 8.879
RRT0.92 - 9.142
RRT0.95 - 9.385
PAR-101 - 9.973
RRT1.03 - 10.393
RRT1.07 - 10.689
RRT1.10 - 10.996
RRT1.13 - 11.217
Peak 8F - 11.543
RRT1.230 - 11.943
peak8E - 12.306

FIGURE 3

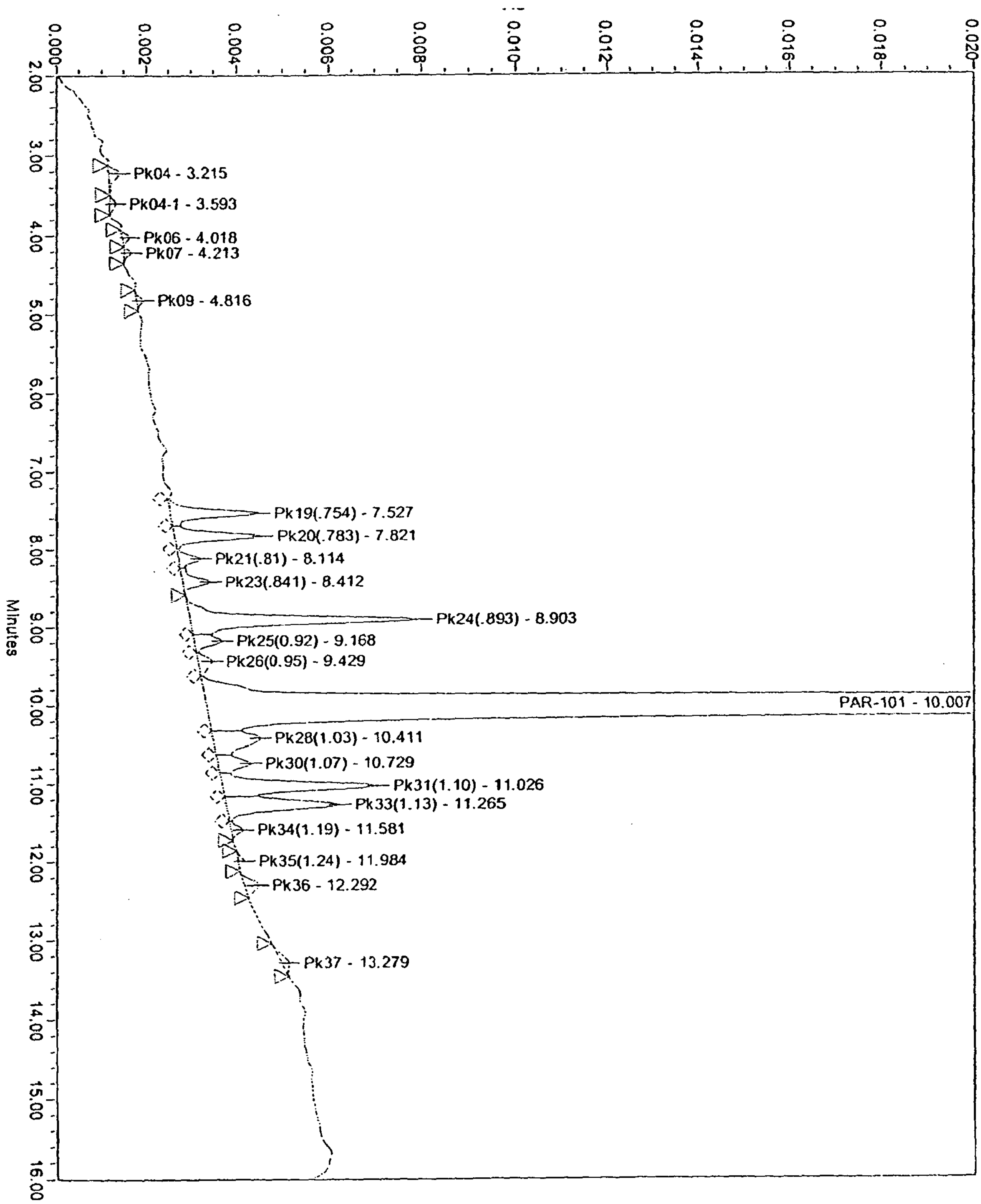
0.000
0.002
0.004
0.006
0.008
0.010
0.012
0.014
0.016
0.018
0.020
2.00
3.00
4.00
5.00
6.00
7.00
8.00
9.00
10.00
11.00
12.00
13.00
14.00
15.00
16.00
Minutes
Pk04 - 3.215
Pk04-1 - 3.593
Pk06 - 4.018
Pk07 - 4.213
Pk09 - 4.816
Pk19(.754) - 7.527
Pk20(.783) - 7.821
Pk21(.81) - 8.114
Pk23(.841) - 8.412
Pk24(.893) - 8.903
Pk25(0.92) - 9.168
Pk26(0.95) - 9.429
PAR-101 - 10.007
Pk28(1.03) - 10.411
Pk30(1.07) - 10.729
Pk31(1.10) - 11.026
Pk33(1.13) - 11.265
Pk34(1.19) - 11.581
Pk35(1.24) - 11.984
Pk36 - 12.292
Pk37 - 13.279

FIGURE 4

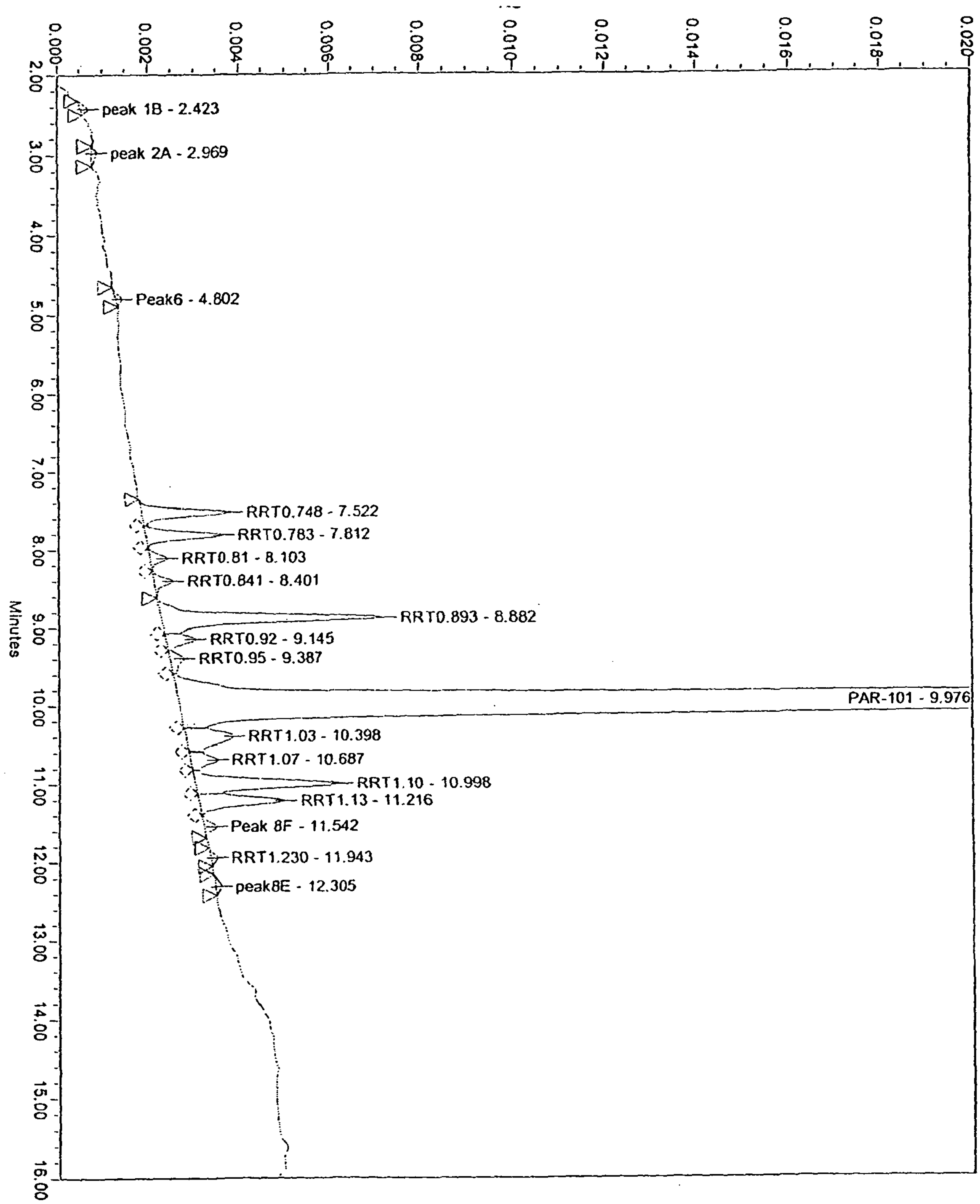
peak 1B - 2.423
peak 2A - 2.969
Peak6 - 4.802
RRT0.748 - 7.522
RRT0.783 - 7.812
RRT0.81 - 8.103
RRT0.841 - 8.401
RRT0.893 - 8.882
RRT0.92 - 9.145
RRT0.95 - 9.387
PAR-101 - 9.976
RRT1.03 - 10.398
RRT1.07 - 10.687
RRT1.10 - 10.998
RRT1.13 - 11.216
Peak 8F - 11.542
RRT1.230 - 11.943
peak8E - 12.305
Minutes
2.00
3.00
4.00
5.00
6.00
7.00
8.00
9.00
10.00
11.00
12.00
13.00
14.00
15.00
16.00
0.000
0.002
0.004
0.006
0.008
0.010
0.012
0.014
0.016
0.018
0.020

FIGURE 5

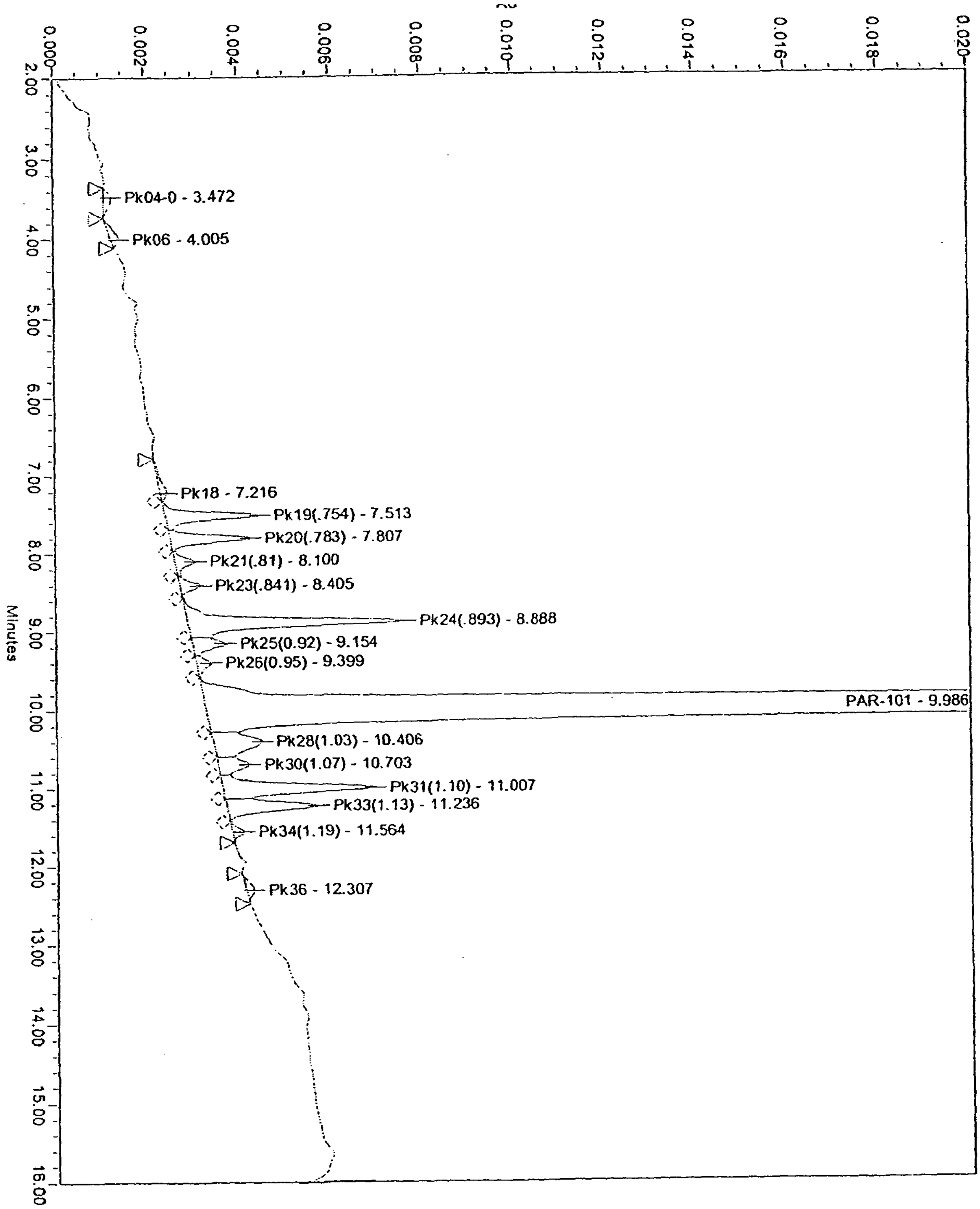

0.000
0.002
0.004
0.006
0.008
0.010
0.012
0.014
0.016
0.018
0.020
2.00
3.00
4.00
5.00
6.00
7.00
8.00
9.00
10.00
11.00
12.00
13.00
14.00
15.00
16.00
Minutes
Pk04-0 - 3.472
Pk06 - 4.005
Pk18 - 7.216
Pk19(.754) - 7.513
Pk20(.783) - 7.807
Pk21(.81) - 8.100
Pk23(.841) - 8.405
Pk24(.893) - 8.888
Pk25(0.92) - 9.154
Pk26(0.95) - 9.399
PAR-101 - 9.986
Pk28(1.03) - 10.406
Pk30(1.07) - 10.703
Pk31(1.10) - 11.007
Pk33(1.13) - 11.236
Pk34(1.19) - 11.564
Pk36 - 12.307

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4918174 A
- WO 2006085838 A
- WO 2005112990 A
- US 20060100164 A
- US 20060257981 A
- US 5583115 A
- US 5767096 A
- US 20060269485 A1
- US 20060257981 A1


### Non-patent literature cited in the description


- *J. Antibiotics,* 1987, 575-888
- **SWANSON et al.** In vitro and in vivo evaluation of tiacumicins B and C against Clostridium difficile. *Antimicrobial Agents and Chemotherapy,* June 1991, 1108-1111
- **REVILL et al.** *Drugs of the Future,* 2006, vol. 31 (6), 494-497
- The Handbook of Pharmaceutical Excipients. American Pharmaceutical Association
- Modified-Release Drug Delivery Technology. Marcel Dekker, Inc,
- Remington's Pharmaceutical Sciences. 1990